# FASCICULE DE BREVET EUROPEEN

(11) **EP 3 429 007 B1**
(45) Date de publication et mention de la délivrance du brevet: **27.11.2019**
(21) Numéro de dépôt: 18182683.5
(22) Date de dépôt: 10.07.2018
(51) Int. Cl.: H01M 8/0444, G01N 33/00, C25B 9/10, C25B 15/02, C25B 1/08, C25B 9/06, C25B 9/20, H01M 8/1018, H01M 8/0206, H01M 8/0221, H01M 8/0228, H01M 8/0256

(54) **DISPOSITIF ELECTROCHIMIQUE COMPORTANT UN CAPTEUR D'HYDROGENE**
ELEKTROCHEMISCHE VORRICHTUNG, DIE EINEN WASSERSTOFFSENSOR UMFASST
ELECTROCHEMICAL DEVICE EQUIPPED WITH A HYDROGEN SENSOR

(30) Priorité: 12.07.2017 FR 1756603
(43) Date de publication de la demande: 16.01.2019
(73) Titulaire: Commissariat à l'Énergie Atomique et aux Énergies Alternatives, 75015 Paris (FR)
(72) Inventeur: VERDIN, Baptiste, 38400 SAINT MARTIN D'HERES (FR); VINCENT, Rémi, 38000 GRENOBLE (FR)
(74) Mandataire: GIE Innovation Competence Group

(56) Documents cités:
- EP-A1- 1 296 395
- EP-A2- 1 293 777
- WO-A1-2008/032838

## Description

### DOMAINE TECHNIQUE

Le domaine de l'invention est celui de la détection de la présence d'hydrogène dans un dispositif électrochimique comportant une membrane échangeuse de protons, tel qu'une pile à combustible ou un électrolyseur.

### ÉTAT DE LA TECHNIQUE ANTÉRIEURE

Les dispositifs électrochimiques à membrane électrolytique échangeuse de protons, tels qu'une pile à combustible ou un électrolyseur, comportent habituellement une ou plusieurs cellules électrochimiques ayant chacune un assemblage membrane électrodes (AME), celui-ci étant formé d'une anode et d'une cathode séparées l'une de l'autre par la membrane électrolytique.

Dans le cas d'un électrolyseur, lorsque de l'eau est amenée à l'anode et qu'une différence de potentiel est appliquée aux électrodes, l'oxydation de l'eau est réalisée, produisant ainsi de l'oxygène et des protons. Ces derniers migrent au travers de la membrane électrolytique jusqu'à la cathode où est réalisée une réduction des protons, produisant ainsi de l'hydrogène. Dans le cas d'une pile à combustible, l'anode réalise l'oxydation de l'hydrogène introduit, et la cathode produit de l'eau par une réaction de réduction à partir de l'oxygène introduit, et des protons et électrons provenant de l'anode.

Cependant, la membrane électrolytique présente un coefficient non nul de perméation vis-à-vis de l'hydrogène, de sorte que de l'hydrogène peut diffuser par perméation au travers de la membrane jusqu'à l'électrode opposée. Ainsi, dans le cas de l'électrolyseur, de l'hydrogène peut diffuser jusqu'à l'anode et se mélanger à l'oxygène produit, ce qui peut entraîner un risque d'ignition lorsque la fraction volumique d'hydrogène, en l'absence d'eau liquide, devient supérieure à 4% environ. Dans le cas d'une pile à combustible, une forte proportion d'hydrogène à la cathode peut traduire la présence d'une dégradation structurelle de la membrane électrolytique, par exemple un vieillissement de la membrane ou une rupture locale.

Il existe donc un besoin de détecter la présence d'hydrogène ayant diffusé par perméation au travers de la membrane électrolytique échangeuse de protons dans les dispositifs électrochimiques équipés de telles membranes.

Le document EP1296395 porte sur un joint d'étanchéité d'une pile à combustible, disposé de part et d'autre d'un circuit imprimé permettant d'extraire un signal de mesure. Il décrit notamment un capteur d'hydrogène formé d'un pont de palladium et d'un substrat d'alumine.

Le document WO2008/032838 porte sur une pile à combustible dont une cellule électrochimique comporte un capteur d'hydrogène. Celui-ci est situé dans un canal de distribution d'hydrogène, en aval d'une zone de restriction qui est une zone d'accumulation d'impuretés et de concentration d'hydrogène diminuée.

Le document EP1293777 décrit un capteur d'hydrogène formé d'un assemblage membrane électrodes.

### EXPOSÉ DE L'INVENTION

L'invention a pour objectif de remédier au moins en partie aux inconvénients de l'art antérieur, et plus particulièrement de proposer un dispositif électrochimique à membrane électrolytique échangeuse de proton comportant un capteur d'hydrogène.

Pour cela, l'objet de l'invention est un dispositif électrochimique comportant au moins une cellule électrochimique, laquelle comporte : un assemblage membrane électrodes formé d'une membrane électrolytique échangeuses de protons, d'une première électrode au contact d'une première face de la membrane, et d'une deuxième électrode au contact d'une deuxième face opposée de la membrane ; et deux plaques bipolaires, entre lesquelles est situé l'assemblage membrane électrodes, traversées par au moins un premier collecteur d'évacuation en communication fluidique avec la première face de la membrane ; l'assemblage membrane électrodes comportant une zone active délimitée par les première et deuxième électrodes, et une zone de liaison située entre la zone active et le premier collecteur d'évacuation.

Selon l'invention, le dispositif électrochimique comporte au moins un capteur d'hydrogène, comportant : une anode positionnée dans la zone de liaison au contact de la première face et incluant un catalyseur adapté à assurer l'oxydation de l'hydrogène, et une cathode au contact de la deuxième face et située en regard de l'anode ; une source de tension adaptée à appliquer une tension électrique entre l'anode et la cathode par un circuit électrique ; un capteur de courant, connecté à la source de tension, adapté à mesurer le courant électrique circulant dans le circuit électrique ; une unité de calcul, connectée au capteur de courant, adaptée à détecter la présence d'hydrogène au niveau de la première face à partir de la valeur mesurée du courant électrique.

Certains aspects préférés mais non limitatifs de ce dispositif électrochimique sont les suivants.

L'unité de calcul peut être adaptée à calculer la quantité d'hydrogène oxydé à l'anode à partir de la valeur mesurée du courant électrique.

L'unité de calcul peut être adaptée à calculer la quantité d'hydrogène circulant dans la zone de liaison au niveau de la première face, à partir de la valeur mesurée du courant électrique.

Une première plaque bipolaire peut comporter un premier circuit de distribution fluidique en communication avec ledit premier collecteur d'évacuation et comportant au moins un canal de distribution superposé à l'anode.

Chaque plaque bipolaire peut être formée en au moins une tôle réalisée en un matériau électriquement conducteur.

L'anode et la cathode peuvent être électriquement isolées des plaques bipolaires.

La membrane électrolytique peut comporter une portion en saillie située dans une bordure de la zone de liaison, la source de tension comportant une première piste conductrice solidarisée à la première face de la membrane et reliant l'anode à la portion en saillie, et une deuxième piste conductrice solidarisée à la deuxième face et reliant la cathode à la portion en saillie.

Les première et deuxième pistes conductrices peuvent présenter une épaisseur inférieure ou égale à 10µm.

Une première et une deuxième plaques bipolaires peuvent être formées chacune en une plaque réalisée en un matériau électriquement isolant, et comportant des lignes conductrices agencées de manière à polariser, respectivement, la première électrode indépendamment de l'anode, et la deuxième électrode indépendamment de la cathode.

La première plaque bipolaire peut être réalisée d'un seul tenant, et présente une face interne et une face externe, la face interne présentant des structurations formant un circuit de distribution fluidique, la première plaque bipolaire étant adaptée à appliquer un premier potentiel électrique à la première électrode et un deuxième potentiel électrique à l'anode différent du premier potentiel.

Le matériau isolant de la première plaque bipolaire peut définir la face interne et la face externe opposée, la face interne comportant des canaux de distribution fluidique séparés longitudinalement deux à deux par une paroi longitudinale venant au contact de la première électrode ou de l'anode par une surface d'appui, des lignes électriquement conductrices s'étendant au niveau des surfaces d'appui de parois longitudinales et étant adaptées, pour un premier ensemble d'entre elles, à appliquer à la première électrode un potentiel électrique et, pour un deuxième ensemble d'entre elles, à appliquer à l'anode un potentiel électrique différent.

La première plaque bipolaire peut comporter une première et une deuxième lignes conductrices dites de reprise de contact s'étendant sur la face externe, et des premiers et deuxièmes vias conducteurs s'étendant entre la face externe et la face interne dans les parois longitudinales, la première ligne conductrice de reprise de contact étant adaptée à appliquer un potentiel électrique au premier ensemble de lignes conductrices par des vias conducteurs, et la deuxième ligne conductrice de reprise de contact étant adaptée à appliquer un potentiel électrique différent au deuxième ensemble de lignes conductrices par les autres vias conducteurs.

### BRÈVE DESCRIPTION DES DESSINS

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels :
la figure 1 est une vue éclatée et en perspective d'un exemple de dispositif électrochimique selon l'art antérieur, comportant un empilement de cellules électrochimiques ayant chacune un assemblage membrane électrodes (AME) ;
la figure 2 est une vue schématique en coupe longitudinale d'un dispositif électrochimique selon un mode de réalisation, comportant un capteur d'hydrogène ayant des électrodes situées dans une zone de liaison de l'assemblage membrane électrodes ;
Les figures 3A à 3E sont des vues schématiques de dessus de différentes parties d'un dispositif électrochimique selon une première variante du mode de réalisation, dans laquelle les plaques bipolaires sont de type tôles conductrices :

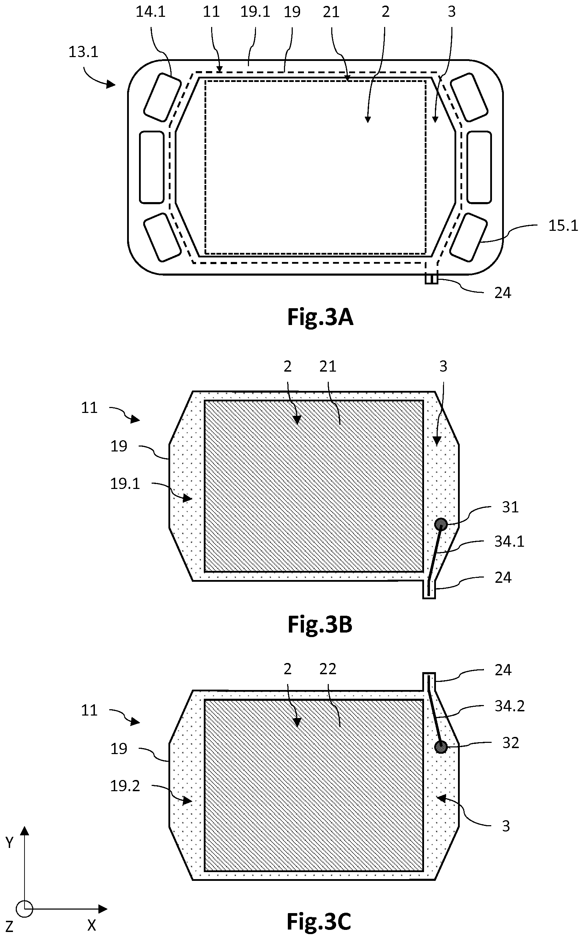

   - la fig.3A illustre une plaque bipolaire recouvrant un AME ;
   - les fig.3B et 3C illustrent un AME, du côté de la première électrode (fig.3B) et du côté de la deuxième électrode (fig.3C), dont la membrane comporte une saillie jusqu'à laquelle s'étend une piste conductrice en contact avec l'anode ou la cathode de détection ;

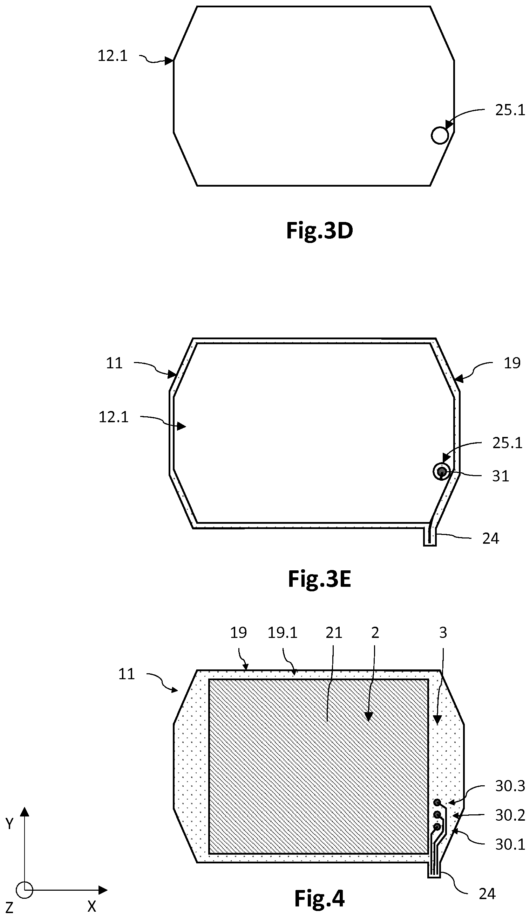

   - les fig.3D et 3E illustrent une couche de diffusion ayant un orifice traversant (fig.3D), celle-ci recouvrant la première électrode de la zone active, de sorte que l'orifice traversant soit positionné en regard de l'anode de détection (fig.3E) ;
La figure 4 est une vue schématique de dessus de l'AME selon une autre variante du mode de réalisation, comportant une pluralité de capteurs d'hydrogène positionnés en sortie d'un ou plusieurs canaux de distribution fluidique ;

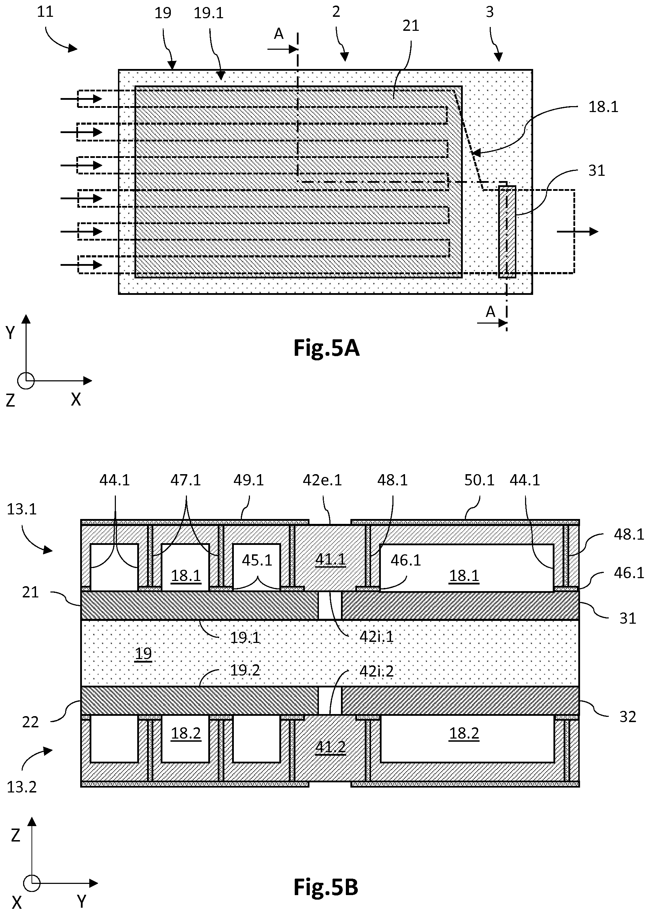

Les figures 5A et 5B sont des vues schématiques d'un dispositif électrochimique selon une deuxième variante du mode de réalisation, dans laquelle les plaques bipolaires sont de type circuit imprimé PCB (pour *Printed Circuit Board,* en anglais) :
   - la fig.5A illustre, en vue de dessus, du côté de la première électrode, un AME recouvert par des canaux de distribution ;
   - la fig.5B illustre une partie du dispositif électrochimique en coupe transversale selon le plan A-A illustré sur la fig.5A.

### EXPOSÉ DÉTAILLÉ DE MODES DE RÉALISATION PARTICULIERS

Sur les figures et dans la suite de la description, les mêmes références représentent les éléments identiques ou similaires. De plus, les différents éléments ne sont pas représentés à l'échelle de manière à privilégier la clarté des figures. Par ailleurs, les différents modes de réalisation et variantes ne sont pas exclusifs les uns des autres et peuvent être combinés entre eux. Sauf indication contraire, les termes « sensiblement », « environ », « de l'ordre de » signifient à 10% près.

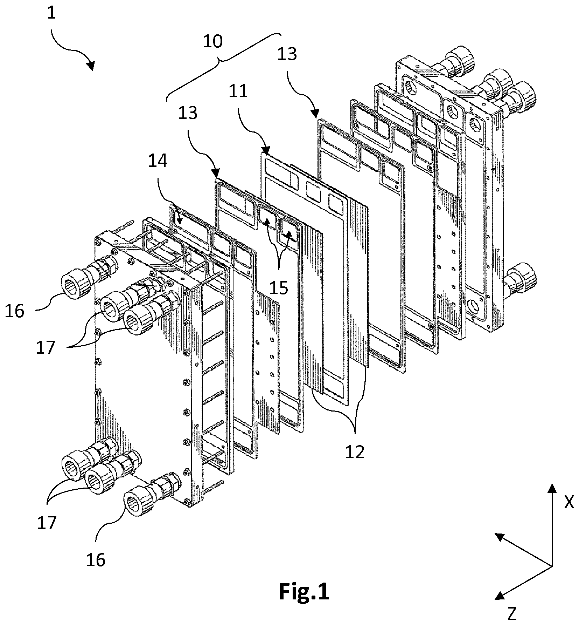

La figure 1 illustre un exemple de dispositif électrochimique 1 selon l'art antérieur, ici tel que décrit dans le document WO2004/102710, comportant un empilement de cellules électrochimiques 10 à membrane électrolytique échangeuse de protons.

On définit ici et pour la suite de la description un repère direct tridimensionnel orthogonal (X,Y,Z), où les axes X et Y sont orientés suivant le plan de la membrane électrolytique de chaque cellule électrochimique 10, l'axe X étant orienté suivant la direction principale d'écoulement fluidique anodique, et où l'axe Z est orienté de manière orthogonale au plan XY.

Chaque cellule électrochimique 10 comporte un assemblage membrane électrodes 11 (AME) formé d'une première électrode, par exemple une anode dans le cas d'un électrolyseur, et d'une deuxième électrode, par exemple une cathode, séparées l'une de l'autre par une membrane électrolytique échangeuse de protons. L'anode, la membrane et la cathode sont des éléments classiques connus de l'homme du métier. Chaque AME 11 s'étend suivant un plan principal de la membrane électrolytique parallèle au plan XY. Des couches de diffusion gazeuse 12 (GDL, pour *Gas Diffusion Layer,* en anglais) recouvrent ici les électrodes.

Chaque AME 11 est séparé de celui des cellules adjacentes par des plaques bipolaires 13. Chaque plaque bipolaire 13 comporte une face destinée à être partiellement au contact de la première électrode d'une cellule 10, et une autre face opposée destinée à être partiellement au contact de la deuxième électrode de la cellule 10 adjacente. Chaque plaque bipolaire 13 est adaptée à amener les espèces réactives à l'anode d'une première cellule 10 d'une part et à la cathode d'une cellule 10 adjacente d'autre part, au moyen de circuits de distribution fluidique, et à évacuer les produits issus des réactions électrochimiques et les espèces non réactives par ces mêmes circuits de distribution, ainsi qu'à transmettre le courant électrique entre les cellules 10. Elle peut également assurer, notamment dans le cas des piles à combustible, l'écoulement d'un fluide caloporteur entre les cellules 10 de manière à permettre l'évacuation de la chaleur produite.

Le dispositif électrochimique 1 comporte également des collecteurs d'écoulement 14, 15 distincts, formés chacun d'une ouverture qui traverse l'empilement de cellules 10, et plus précisément qui traverse les plaques bipolaires 13 en regard d'une bordure de l'AME 11. Des collecteurs d'injection 14 assurent l'injection de fluides réactifs à partir de ports d'injection 16, et des collecteurs d'évacuation 15 permettent l'évacuation des produits des réactions électrochimiques jusqu'à des ports d'évacuation 17.

Ainsi, d'une manière générale, chaque cellule électrochimique 10 comporte un premier circuit de distribution reliant un premier collecteur d'injection à un premier collecteur d'évacuation, adapté à amener le fluide réactif entrant F_{1,in} au contact de la première électrode. Un deuxième circuit de distribution relie un deuxième collecteur d'injection à un deuxième collecteur d'évacuation, adapté à amener un autre fluide réactif entrant F_{2,in} au contact de la deuxième électrode.

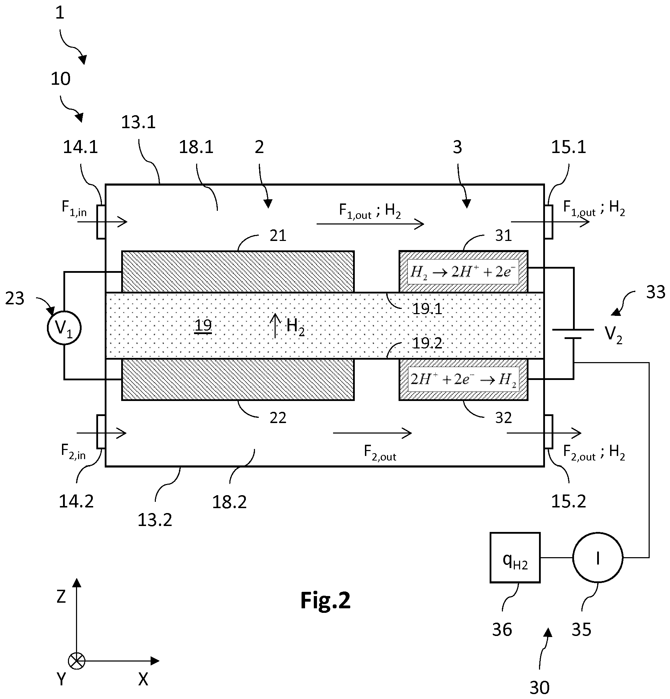

La figure 2 illustre de manière schématique, en coupe longitudinale, un dispositif électrochimique 1 selon un mode de réalisation comportant une cellule électrochimique 10 équipée d'un capteur d'hydrogène 30.

La cellule électrochimique 10 comporte deux plaques bipolaires 13.1, 13.2 entre lesquelles est situé un AME 11. Chaque plaque bipolaire 13.1, 13.2 comporte un circuit de distribution 18.1, 18.2 d'un fluide adapté à assurer l'écoulement d'un fluide au niveau d'une électrode 21, 22 de l'AME 11 entre deux collecteurs d'écoulement, plus précisément entre un collecteur d'injection 14.1, 14.2 et un collecteur d'évacuation 15.1, 15.2. Le circuit de distribution 18.1, 18.2 est formé d'un réseau de canaux de distribution qui s'étendent entre une entrée communiquant avec le collecteur d'injection 14.1, 14.2 et une sortie communiquant avec le collecteur d'évacuation 15.1, 15.2. La première plaque bipolaire 13.1 permet d'amener un fluide réactif à une première électrode 21 de l'AME 11, et la deuxième plaque bipolaire 13.2 permet l'écoulement d'un autre fluide vers et/ou à partir de la deuxième électrode 22.

L'assemblage membrane électrodes 11 comporte une même membrane électrolytique 19 échangeuse de protons séparant d'une part la première électrode 21 de la deuxième électrode 22 situées en regard l'une de l'autre, et d'autre part une anode 31 d'une cathode 32 du capteur de détection 30 situées en regard l'une de l'autre.

Les première et deuxième électrodes 21, 22 délimitent la zone active 2 de l'AME 11. Aussi, l'AME 11 comporte la zone active 2 ainsi qu'une zone dite de liaison 3, cette dernière étant située entre la zone active 2 et un collecteur d'évacuation 15.1, et plus précisément entre la zone active 2 et le premier collecteur d'évacuation 15.1. Ainsi, la membrane électrolytique 19 est revêtue des première et deuxième électrodes 21, 22 dans la zone active 2 uniquement et non pas dans la zone de liaison 3. Dans la zone active 2 ont lieu des réactions électrochimiques participant au rendement électrochimique du dispositif 1 : la zone active 2 est ainsi le lieu de l'électrolyse de l'eau dans le cas d'un électrolyseur, et le lieu de la génération d'eau par réaction de l'hydrogène et de l'oxygène dans le cas d'une pile à combustible. Aussi, la zone de liaison 3 peut être dite inactive dans le sens où elle ne participe pas au rendement électrochimique du dispositif.

La membrane électrolytique 19 permet la diffusion de protons d'une anode jusqu'à une cathode, l'anode et la cathode étant situées en regard l'une de l'autre, les protons pouvant se présenter au sein de la membrane sous la forme d'ions H₃O⁺. Elle présente un coefficient non nul de perméation de l'hydrogène, autorisant ainsi la diffusion de l'hydrogène au travers de la membrane de la cathode jusqu'à l'anode. La membrane électrolytique 19 est monolithique, et est réalisée en un même matériau sur toute son étendue surfacique. Elle peut ainsi être réalisée à partir de matériaux choisis habituellement par l'homme du métier, tels que ceux commercialisés sous la référence Nafion 115 ou Nafion 117 dans le cas d'un électrolyseur qui présente un coefficient de perméation à l'hydrogène de l'ordre de 1,25.10⁴ cm³/s/cm² à 80°C et à pression atmosphérique, ou ceux commercialisés sous la référence Nafion 211 dans le cas d'une pile à combustible.

La première électrode 21 est au contact de la première face 19.1 de la membrane électrolytique 19 et reçoit un fluide réactif F_{1,in} par le premier circuit de distribution 18.1, à savoir de l'oxygène dans le cas d'une pile à combustible ou de l'eau dans le cas d'un électrolyseur. Elle comporte une couche active adaptée à réaliser une réaction d'oxydation ou de réduction du fluide entrant F_{1,in}, la couche active ayant un catalyseur favorisant cette réaction électrochimique.

La deuxième électrode 22 est au contact de la deuxième face 19.2 opposée de la membrane électrolytique 19, et reçoit de l'hydrogène à oxyder dans le cas d'une pile à combustible, ou génère de l'hydrogène dans le cas d'un électrolyseur. Elle comporte une couche active adaptée à réaliser une réaction de réduction ou d'oxydation, la couche active ayant un catalyseur favorisant cette réaction électrochimique.

Les première et deuxième électrodes 21, 22 sont électriquement connectées l'une à l'autre par un circuit électrique 23 qui autorise la circulation des électrons entre les deux électrodes 21, 22 et permet, dans le cas d'un électrolyseur, l'application d'une différence de potentiel électrique entre les première et deuxième électrodes 21, 22. Dans ce cas, la tension électrique appliquée V1 est positive, dans le sens où le potentiel électrique imposé à la première électrode 21 est supérieur à celui imposé à la deuxième électrode 22. Elle peut être comprise entre 1,3V et 3V, par exemple égale à 1,8V environ, pour une densité de courant comprise, par exemple, entre 50mA/cm² et 4A/cm². Dans le cas d'une pile à combustible, le circuit électrique 23 peut comporter une charge électrique aux bornes de laquelle une différence de potentiel électrique est appliquée par les électrodes 21, 22.

Dans le cas d'une pile à combustible, la première électrode 21 est une cathode assurant une réaction de réduction produisant de l'eau à partir d'oxygène introduit, et de protons et d'électrons provenant de la deuxième électrode 22. Et la deuxième électrode 22 est une anode assurant une réaction d'oxydation de l'hydrogène introduit. Du fait de la perméation de la membrane électrolytique 19, de l'hydrogène peut diffuser à partir de la deuxième face 19.2 jusqu'à la première face 19.1 de la membrane électrolytique 19, et s'écouler dans le premier circuit de distribution cathodique 18.1.

Dans le cas d'un électrolyseur, la première électrode 21 est une anode assurant une rédaction d'oxydation de l'eau introduite, et la deuxième électrode 22 est une cathode assurant la réduction des protons. Du fait de la perméation de la membrane électrolytique 19, de l'hydrogène peut également diffuser à partir de la deuxième face 19.2 jusqu'à la première face 19.1, et s'écouler dans le premier circuit de distribution 18.1.

Le capteur d'hydrogène 30 comporte une anode 31 et une cathode 32 de détection, toutes deux situées dans la zone de liaison 3, entre la zone active 2 et le collecteur d'évacuation 15.1, en aval de la première électrode 21 dans le sens longitudinal d'écoulement fluidique depuis le collecteur d'injection 14.1 vers le collecteur d'évacuation 15.1. Le capteur d'hydrogène 30 est adapté à détecter la présence d'hydrogène dans le premier circuit de distribution 18.1 par oxydation d'au moins une partie de l'hydrogène présent. L'anode 31 et la cathode 32 sont séparées l'une de l'autre par la même membrane électrolytique 19, et le capteur 30 comporte en outre une source de tension 33 permettant d'appliquer une différence de potentiel V2 entre l'anode 31 et la cathode 32.

L'anode 31 de détection est au contact de la première face 19.1 de la membrane électrolytique 19 et est située dans la zone de liaison 3 de l'AME 11 de sorte qu'elle est accessible par le fluide F_{1,out} circulant dans le premier circuit de distribution 18.1. Elle est distincte de la première électrode 21 dans le sens où elle en est électriquement isolée. Elle comporte une couche active adaptée à réaliser l'oxydation de l'hydrogène, celle-ci comportant un catalyseur favorisant cette réaction d'oxydation, par exemple des particules de platine supportées par du carbone, voire du palladium. La réaction d'oxydation de l'hydrogène s'écrit :

*H*₂ → 2*H*⁺ + 2*e⁻*

La cathode 32 de détection est au contact de la deuxième face 19.2 de la membrane électrolytique 19 et est en regard de l'anode 31 de détection, en étant en communication fluidique avec le deuxième circuit de distribution 18.2. Elle est distincte de la deuxième électrode 22 dans le sens où elle en est électriquement isolée. Elle comporte une couche active adaptée à réaliser la réduction des protons ayant diffusé au travers de la membrane électrolytique 19 avec les électrons issus de l'oxydation de l'hydrogène. La couche active comporte un catalyseur favorisant cette réaction de réduction, par exemple des particules de platine supportées par du carbone, voire du palladium. La réaction de réduction des protons s'écrit :

2*H*⁺ + *2e⁻* → *H*₂

Le capteur d'hydrogène 30 comporte une source de tension électrique 33 permettant d'appliquer une différence de potentiel électrique V2, de préférence continue, entre l'anode 31 et la cathode 32 de détection, permettant ainsi l'oxydation d'au moins une partie de l'hydrogène présent à l'anode 31, la circulation des électrons jusqu'à la cathode 32, puis la réduction des protons à la cathode 32. A titre d'exemple, la tension électrique appliquée V2 est de même signe que la différence de potentiels électriques entre les électrodes 21, 22 dans le cas d'un électrolyseur ou d'une pile à combustible. D'une manière générale, elle présente une valeur inférieure, en valeur absolue, à la tension électrique V1 et peut être égale à 0,2V environ, voire à 0,4V environ. La source de tension 33 comporte ainsi un générateur de tension associé à un circuit électrique formé de pistes conductrices reliant le générateur de tension à l'anode 31 et à la cathode 32.

Le capteur d'hydrogène 30 comporte un capteur de courant électrique 35, connecté à la source de tension 33. Le capteur de courant 35 mesure la valeur du courant circulant ou non dans le circuit électrique de la source de tension 33, selon que de l'hydrogène, initialement présent ou non dans le premier circuit de distribution 18.1 est oxydé à l'anode 31.

Le capteur d'hydrogène 30 comporte également une unité de calcul 36, connectée au capteur de courant 35, qui est adaptée à détecter la présence d'hydrogène au niveau de la première face 19.1 de la membrane électrolytique 19, c'est-à-dire dans le premier circuit de distribution 18.1. A partir de la valeur mesurée du courant électrique, l'unité de calcul 36 peut être en mesure de calculer la quantité d'hydrogène oxydé à l'anode 31. De manière avantageuse, l'unité de calcul 36 intègre une base de données (abaque obtenue préalablement) ou un modèle électrochimique permettant d'estimer, à partir de la valeur mesurée du courant électrique, la quantité d'hydrogène circulant dans la zone de liaison 3 au niveau de la première face 19.1 de la membrane électrolytique 19, c'est-à-dire dans le premier circuit de distribution 18.1. La base de données peut avoir été obtenue préalablement, par exemple en reliant, pour un point de la courbe de polarisation de la cellule électrochimique 10, la quantité d'hydrogène circulant dans le premier circuit de distribution 18.1 en fonction de la valeur du courant électrique fournie par le capteur 35.

Ainsi, le dispositif électrochimique 1 comporte un capteur d'hydrogène 30 intégré au sein de la cellule électrochimique 20, dans le sens où il permet de détecter la présence éventuelle d'hydrogène dans le premier circuit de distribution 18.1, c'est-à-dire du côté 19.1 de la membrane électrolytique 19 où est susceptible d'avoir diffusé de l'hydrogène par perméation.

Le capteur d'hydrogène 30 permet ainsi de détecter en temps réel la présence d'hydrogène ayant diffusé par perméation au travers de la membrane électrolytique 19, voire même de fournir la quantité d'hydrogène oxydé à l'anode 31 de détection. Il permet avantageusement d'estimer la quantité d'hydrogène circulant dans le premier circuit de distribution 18.1, en sortie de la zone active 2. Ainsi, cette information peut être exploitée pour limiter les risques d'ignition dans le cas d'un électrolyseur et/ou pour connaître l'état de santé de la membrane électrolytique 19 (degré de vieillissement, rupture locale, etc...).

On évite ainsi la nécessité d'avoir recours à une instrumentation spécifique de la cellule électrochimique 20 qui peut être difficile ou coûteuse à mettre en œuvre. Le capteur d'hydrogène 30 présente en outre l'avantage de diminuer la proportion volumique d'hydrogène dans le fluide sortant F_{1,out} qui circule dans le premier circuit de distribution 18.1, par le fait même de détecter l'hydrogène présent par oxydation.

Le fonctionnement du dispositif électrochimique 1 est maintenant décrit dans le cas d'un électrolyseur.

De l'eau F_{1,in} est injectée à l'entrée du premier circuit de distribution 18.1 par le collecteur d'injection 14.1, et vient au contact de la première électrode 21, qui est ici une anode. Dans cet exemple, de l'eau F_{2,in} peut également être injectée à l'entrée du deuxième circuit de distribution 18.2 par le deuxième collecteur d'injection 14.2. L'eau est oxydée à la première électrode 21 de la zone active 2, ce qui génère de l'oxygène circulant dans le premier circuit de distribution 18.1, et les protons sont réduits à la deuxième électrode 22, générant ainsi de l'hydrogène circulant dans le deuxième circuit de distribution 18.2.

Cependant, de l'hydrogène généré à la deuxième électrode 22 diffuse par perméation au travers de la membrane électrolytique 19 jusqu'à la première électrode 21. En sortie de la zone active 2, le fluide F_{1,out} circulant dans le premier circuit de distribution 18.1 comporte ainsi de l'oxygène ainsi qu'une proportion volumique non nulle d'hydrogène.

Le fluide sortant F_{1,out} traverse ensuite la zone de liaison 3, située entre la zone active 2 et le premier collecteur d'évacuation 15.1. Il vient ainsi au contact de l'anode 31 de détection, qui réalise alors l'oxydation d'au moins une partie de l'hydrogène présent, une tension électrique V2 non nulle étant appliquée entre les électrodes 31, 32. Les protons diffusent alors jusqu'à la cathode 32 et les électrons circulent dans le circuit électrique de la source de tension 33. La réduction des protons est alors effectuée à la cathode 32.

Le capteur de courant 35 mesure ainsi une valeur non nulle du courant électrique circulant dans la source de tension 33, et l'unité de calcul 36 détecte la présence d'hydrogène lorsque la valeur mesurée est non nulle. L'unité 36 peut également calculer la quantité d'hydrogène ayant été oxydé à l'anode 31. L'unité de calcul 36 peut estimer en outre la quantité d'hydrogène circulant dans le premier circuit de distribution 18.1 en sortie de la zone active 2 à partir de la valeur mesurée du courant électrique.

Les figures 3A à 3E illustrent en vue de dessus différentes parties du dispositif électrochimique 1 selon une première variante du mode de réalisation.

Dans cette variante, les plaques bipolaires 13 sont formées de tôles réalisées en un matériau électriquement conducteur, dont les circuits de distribution 18 peuvent être obtenus par emboutissage ou moulage. Elles autorisent ainsi la circulation électrique entre les première et deuxième électrodes 21, 22. L'anode 31 et la cathode 32 de détection sont alors électriquement isolées des plaques bipolaires 13 mais en communication fluidique avec les circuits de distribution 18 de ces dernières.

La figure 3A est une vue de dessus d'une première plaque bipolaire 13.1 en communication fluidique avec la première face 19.1 de la membrane électrolytique 19. Elle comporte dans cet exemple trois collecteurs d'injection : un premier collecteur 14.1 pour le fluide entrant F_{1,in} destiné à venir au contact de la première électrode 21, un deuxième collecteur pour le fluide entrant F_{2,in} destiné à venir au contact de la deuxième électrode, et ici un troisième collecteur pour l'injection d'un fluide caloporteur destiné à circuler dans un circuit de refroidissement, dans le cas d'une pile à combustible. Elle comporte en outre trois collecteurs d'évacuation : un premier collecteur 15.1 pour recevoir le fluide sortant F_{1,out} circulant dans le premier circuit de distribution 18.1, un deuxième collecteur pour recevoir celui F_{2,out} circulant dans le deuxième circuit de distribution, et un troisième collecteur pour le fluide caloporteur. La zone active 2 de l'AME 11 est illustrée par des traits pointillés, ainsi que la membrane électrolytique 19. Ainsi, la zone de liaison 3 de l'AME 11 est située entre la zone active 2 et le premier collecteur d'évacuation 15.1. Comme décrit en détail ensuite, la membrane électrolytique 19 comporte une portion en saillie 24 qui déborde le contour de la plaque bipolaire 13.1.

Les figures 3B et 3C sont des vues de dessus d'une AME 11, du côté de la première électrode 21 pour la fig.3B et du côté de la deuxième électrode 22 pour la fig.3C. L'anode 31 est au contact de la première face 19.1 de la membrane électrolytique 19 et positionnée dans la zone de liaison 3, et la cathode 32 est au contact de la deuxième face 19.2 et en regard de l'anode 31. L'anode 31 comme la cathode 32 sont distinctes, et donc électriquement isolées, respectivement, de la première électrode 21 et de la deuxième électrode 22.

La membrane électrolytique 19 comporte une portion 24 en saillie située au niveau d'une bordure de la zone de liaison 3. La portion 24 en saillie présente une dimension telle qu'elle dépasse le contour des plaques bipolaires 13, et est donc accessible à partir de l'extérieur de l'empilement de cellules électrochimiques.

La source de courant comporte une première piste conductrice 34.1 qui s'étend continûment sur la première face 19.1 à partir de l'anode 31 avec laquelle elle est en contact électrique jusqu'à la portion 24 en saillie. Elle comporte une deuxième piste conductrice 34.2 qui s'étend continûment sur la deuxième face 19.2 à partir de la cathode 32 avec laquelle elle est en contact électrique jusqu'à la portion 24 en saillie. Les pistes conductrices 34.1, 34.2 peuvent prendre la forme d'un circuit imprimé, par exemple réalisé par dépôt d'une encre formée d'un matériau conducteur et d'un ionomère, présentant avantageusement une épaisseur inférieure ou égale à 10µm. On limite ainsi les surépaisseurs locales qui peuvent induire des défauts d'étanchéité ou des inhomogénéités de contraintes mécaniques. Ainsi, le générateur de tension peut être facilement connecté aux pistes conductrices 34.1, 34.2 dans la mesure où elles débordent le contour des plaques bipolaires.

Les figures 3D et 3E sont des vues de dessus d'une couche de diffusion 12.1 (GDL, pour *Gas Diffusion Layer,* en anglais) comportant un orifice traversant 25.1. La première couche de diffusion 12.1 est destinée à venir au contact de la première face 19.1, et à recouvrir la zone active 2 et la zone de liaison 3. Pour éviter que la couche de diffusion 12.1 ne soit au contact de l'anode 31 de détection, un orifice traversant 25.1 est prévu, dont le contour est dimensionné pour éviter tout contact avec la bordure de l'anode 31. Ainsi, on évite le contact électrique entre la couche de diffusion 12.1 et l'anode 31 tout en autorisant le fluide sortant F_{1,out} de rejoindre cette dernière. Il en est de même pour une deuxième couche de diffusion vis-à-vis de la cathode. Les pistes conductrices sont également isolées électriquement des couches de diffusion, par exemple par un film isolant (non représenté) formant un renfort de l'AME.

De préférence, un film polymère (non représenté) électriquement isolant mais poreux peut être disposé entre l'anode 31 et la première plaque bipolaire 13.1. Le film peut être un polymère tel que commercialisé par la société Celgard, par exemple le Celgard 2500. Ainsi, l'isolation électrique entre l'anode 31 et la première plaque bipolaire 13.1 est renforcée, tout en préservant la communication fluidique entre le premier circuit de distribution 18.1 et l'anode 31. Il en est de même pour la cathode 32 vis-à-vis de la deuxième plaque bipolaire 18.2.

La figure 4 illustre une vue de dessus d'un AME selon une autre variante, du côté de la première électrode 21, qui se distingue de celle de la fig.3B essentiellement en ce que le dispositif 1 comporte une pluralité de capteurs d'hydrogène 30.1, 30.2, 30.3. Chaque capteur d'hydrogène 30.1, 30.2, 30.3 comporte une anode et une cathode disposées dans la zone de liaison 3. Les anodes de détection des différents capteurs d'hydrogène 30.1, 30.2, 30.3 sont électriquement isolées les unes des autres et placées chacune en regard d'un ou plusieurs canaux du premier circuit de distribution. Les cathodes de détection sont situées en regard des anodes correspondantes. Les capteurs d'hydrogène 30.1, 30.2, 30.3 comportent des première et deuxième pistes conductrices, qui s'étendent jusqu'à une portion 24 en saillie de la membrane électrolytique 19. Plusieurs portions en saillie peuvent être prévues, ou une unique portion en saillie comme représenté ici. Ainsi, il est possible de détecter la présence d'hydrogène de manière localisée, et ainsi d'indiquer si un ou plusieurs canaux de distribution comportent davantage d'hydrogène que les autres. Cela permet notamment d'assurer un suivi plus précis de l'état de santé de la membrane électrolytique 19, et de localiser plus précisément une éventuelle rupture de la membrane. Des microvannes peuvent être prévues, dans le but d'obturer les canaux de distribution au niveau desquels serait située la rupture de la membrane.

Les figures 5A et 5B illustrent, en vue de dessus (fig.5A) et en coupe transversale (fig.5B), différentes parties du dispositif électrochimique 1 selon une deuxième variante du mode de réalisation.

Dans cette variante, les plaques bipolaires 13 comportent une plaque structurée en un matériau électriquement isolant à l'intérieur duquel s'étendent des lignes conductrices adaptées à polariser les première et deuxième électrodes 21, 22 indépendamment des anode 31 et cathode 32 de détection. Ainsi, l'anode 31 et la cathode 32 de détection sont alors au contact du matériau isolant des plaques bipolaires 13 mais électriquement isolées des première et deuxième électrodes 21, 22. Les plaques bipolaires 13 sont alors de type circuit imprimé PCB.

La figure 5A est une vue de dessus d'une AME 11 du côté de la première face 19.1 de la membrane électrolytique 19, et d'un exemple de premier circuit de distribution 18.1 illustré en traits pointillés.

L'AME 11 comporte ainsi une membrane électrolytique 19 dont la première face 19.1 est revêtue par la première électrode 21 qui délimite la zone active 2, et par l'anode 31 de détection située dans la zone de liaison 3. Une pluralité de canaux de distribution parcourt la zone active 2 et, ici à titre purement illustratif, se rejoignent dans la zone de liaison 3 pour former une zone d'homogénéisation de l'écoulement. D'autres agencements des canaux sont bien sûr possibles. Ainsi, à titre d'exemples, les canaux de distribution peuvent rester distincts les uns des autres dans la zone de liaison, voire se rejoindre par groupes.

A la différence de la première variante, dans la mesure où la polarisation de l'anode 31 et de la cathode 32 de détection est assurée par les plaques bipolaires 13, la membrane électrolytique 19 ne comporte pas de portion en saillie recevant des pistes conductrices de la source de courant.

La figure 5B illustre, de manière schématique et partielle, en coupe transversale selon le plan de coupe A-A illustré sur la fig.5A, un exemple de plaques bipolaires 13 de type PCB selon cette deuxième variante.

Est ici illustré l'AME 11, dont une première zone forme la zone active 2 et une deuxième zone, distincte de la première, forme la zone de liaison 3, située en aval de la zone active 2 dans la continuité fluidique du premier circuit de distribution 18.1, en direction du premier collecteur d'évacuation 15.1 (non représenté). La zone active 2 comporte la première électrode 21 et la deuxième électrode 22 séparées l'une de l'autre par la membrane électrolytique 19, et la zone de liaison 3 comporte l'anode 31 et la cathode 32 séparées l'une de l'autre par la même membrane électrolytique 19. L'anode 31 et la première électrode 21 sont distinctes l'une de l'autre pour éviter tout contact électrique, tout comme le sont la cathode 32 et la deuxième électrode 32. De manière connue, la membrane électrolytique 19 est conductrice de protons mais isolante vis-à-vis des électrons.

Les circuits de distribution 18.1, 18.2 sont formés par structuration des plaques bipolaires. Ainsi, le premier circuit de distribution 18.1 est réalisé dans une même plaque bipolaire 13.1 présentant des structurations définissant des canaux de distribution fluidique, laquelle étant adaptée à appliquer un potentiel électrique à la première électrode 21 et un autre potentiel électrique différent à l'anode 31. De même, le deuxième circuit de distribution 18.2 est réalisé dans une même plaque bipolaire 13.2 présentant des structurations définissant des canaux de distribution fluidique, laquelle étant adaptée à appliquer un potentiel électrique à la deuxième électrode 22 et un autre potentiel électrique, éventuellement différent, à la cathode 32.

Dans cet exemple, chaque plaque bipolaire 13.1, 13.2 est réalisée à partir d'un circuit imprimé de type PCB (*Printed Circuit Board,* en anglais). Elle comporte ainsi une partie 41.1, 41.2 en un matériau électriquement isolant, par exemple une céramique, sur et dans lequel s'étendent des lignes électriques.

En référence à la première plaque bipolaire 13.1 (la deuxième plaque bipolaire 13.2 est ici identique), la partie isolante 41.1 présente une face externe 42e.1 et une face interne 42i.1 opposée l'une à l'autre suivant l'axe d'épaisseur de la plaque 13.1. La face interne 42i.1 est orientée vers la première face 19.1 de la membrane électrolytique 19, et comporte des structurations définissant le circuit de distribution 18.1.

Les canaux du circuit de distribution 18.1 sont séparés deux à deux et bordés par une paroi longitudinale 44.1 de la plaque isolante 41.1, dont l'extrémité, formant une surface d'appui au contact de la première électrode 21 ou de l'anode 31, est au moins partiellement revêtue par une piste électrique 45.1, 46.1 dite de polarisation.

Ainsi, tout ou partie des extrémités des parois longitudinales 44.1 séparant et bordant les canaux de distribution est revêtue d'une telle piste électrique 45.1, 46.1 de polarisation. Les pistes de polarisation 45.1 contactent la première électrode 21, et les pistes de polarisation 46.1 contactent l'anode 31. Les pistes de polarisation 45.1 sont électriquement isolées des pistes de polarisation 46.1.

Les pistes de polarisation 45.1, au contact de la première électrode 21, sont reliées à une piste électrique 49.1 dite de reprise de contact qui s'étend sur la face externe 42e.1. La piste 49.1 de reprise de contact est connectée aux pistes de polarisation 45.1 par des premiers vias 47.1. Les vias 47.1 sont des orifices traversants remplis d'un matériau électriquement conducteur, qui s'étendent suivant l'axe d'épaisseur de la plaque isolante 13.1 dans les parois longitudinales 44.1.

De manière similaire, les pistes de polarisation 46.1, au contact de l'anode 31 de détection, sont reliées à une autre piste électrique 50.1 de reprise de contact qui s'étend sur la même face externe 42e.1. La piste 50.1 de reprise de contact est connectée aux pistes de polarisation 46.1 par des vias 48.1. Les première et deuxième pistes électriques 49.1, 50.1 de reprise de contact sont électriquement séparées l'une de l'autre.

Ainsi, la même plaque bipolaire 13.1 forme un premier circuit de distribution fluidique 18.1 pour la première électrode 21 et l'anode 31 de détection, et permet d'appliquer à ces deux électrodes 21, 31 des potentiels électriques différents l'un de l'autre.

La deuxième plaque bipolaire 13.2 peut être identique ou similaire à la première plaque bipolaire 13.1 décrite précédemment. Il est par ailleurs possible d'empiler plusieurs assemblages membrane électrodes suivant l'axe d'épaisseur, les assemblages membrane électrodes adjacents étant séparés par des plaques bipolaires de type PCB similaires à celles illustrées sur la fig.5B.

## Revendications

1. Dispositif électrochimique (1), comportant au moins une cellule électrochimique (10), comportant :
o un assemblage membrane électrodes (11) formé d'une membrane électrolytique (19) échangeuses de protons, d'une première électrode (21) au contact d'une première face (19.1) de la membrane (19), et d'une deuxième électrode (22) au contact d'une deuxième face (19.2) opposée de la membrane (19),
o deux plaques bipolaires (13.1, 13.2), entre lesquelles est situé l'assemblage membrane électrodes (11), traversées par au moins un premier collecteur d'évacuation (15.1) en communication fluidique avec la première face (19.1) de la membrane (19),
o l'assemblage membrane électrodes (11) comportant une zone active (2) délimitée par les première et deuxième électrodes (21, 22), et une zone de liaison (3) située entre la zone active (2) et le premier collecteur d'évacuation (15.1) ;
**caractérisé en ce qu'**il comporte au moins un capteur d'hydrogène (30), comportant :
o une anode (31) positionnée dans la zone de liaison (3) au contact de la première face (19.1) et incluant un catalyseur adapté à assurer l'oxydation de l'hydrogène, et une cathode (32) au contact de la deuxième face (19.2) et située en regard de l'anode (31) ;
o une source de tension (33) adaptée à appliquer une tension électrique entre l'anode (31) et la cathode (32) par un circuit électrique ;
o un capteur de courant (35), connecté à la source de tension (33), adapté à mesurer le courant électrique circulant dans le circuit électrique ;
o une unité de calcul (36), connectée au capteur de courant (35), adaptée à détecter la présence d'hydrogène au niveau de la première face (19.1) à partir de la valeur mesurée du courant électrique.

2. Dispositif électrochimique (1) selon la revendication 1, dans lequel l'unité de calcul (36) est adaptée à calculer la quantité d'hydrogène oxydé à l'anode (31) à partir de la valeur mesurée du courant électrique.

3. Dispositif électrochimique (1) selon la revendication 1 ou 2, dans lequel l'unité de calcul (36) est adaptée à calculer la quantité d'hydrogène circulant dans la zone de liaison (3) au niveau de la première face (19.1), à partir de la valeur mesurée du courant électrique.

4. Dispositif électrochimique (1) selon l'une quelconque des revendications 1 à 3, dans lequel une première plaque bipolaire (13.1) comporte un premier circuit de distribution fluidique (18.1) en communication avec ledit premier collecteur d'évacuation (15.1) et comportant au moins un canal de distribution superposé à l'anode (31).

5. Dispositif électrochimique (1) selon l'une quelconque des revendications 1 à 4, dans lequel chaque plaque bipolaire (13.1, 13.2) est formée en au moins une tôle réalisée en un matériau électriquement conducteur.

6. Dispositif électrochimique (1) selon la revendication 5, dans lequel l'anode (31) et la cathode (32) sont électriquement isolées des plaques bipolaires (13.1, 13.2).

7. Dispositif électrochimique (1) selon la revendication 5 ou 6, dans lequel la membrane électrolytique (19) comporte une portion (24) en saillie située dans une bordure de la zone de liaison (3), la source de tension (33) comportant une première piste conductrice (34.1) solidarisée à la première face (19.1) de la membrane (19) et reliant l'anode (31) à la portion (24) en saillie, et une deuxième piste conductrice (34.2) solidarisée à la deuxième face (19.2) et reliant la cathode (32) à la portion (24) en saillie.

8. Dispositif électrochimique (1) selon la revendication 7, dans lequel les première et deuxième pistes conductrices (34.1, 34.2) présentent une épaisseur inférieure ou égale à 10µm.

9. Dispositif électrochimique (1) selon l'une quelconque des revendications 1 à 4, dans lequel une première et une deuxième plaques bipolaires (13.1, 13.2) sont formées chacune en une plaque (41.1, 41.2) réalisée en un matériau électriquement isolant, et comportant des lignes conductrices (45, 47,49 ;46, 48, 50) agencées de manière à polariser, respectivement, la première électrode (21) indépendamment de l'anode (31), et la deuxième électrode (22) indépendamment de la cathode (32).

10. Dispositif électrochimique (1) selon la revendication 9, dans lequel la première plaque bipolaire (13.1) est réalisée d'un seul tenant, et présente une face interne (42i.1) et une face externe (42e.1), la face interne (42i.1) présentant des structurations formant un circuit de distribution fluidique (18.1), la première plaque bipolaire (13.1) étant adaptée à appliquer un premier potentiel électrique à la première électrode (21) et un deuxième potentiel électrique à l'anode (31) différent du premier potentiel.

11. Dispositif électrochimique (1) selon la revendication 10, dans lequel le matériau isolant (41.1) de la première plaque bipolaire (13.1) définit la face interne (42i.1) et la face externe (42e.1) opposée, la face interne (42i.1) comportant des canaux de distribution fluidique séparés longitudinalement deux à deux par une paroi longitudinale (44.1) venant au contact de la première électrode (21) ou de l'anode (31) par une surface d'appui, des lignes électriquement conductrices (45.1, 46.1) s'étendant au niveau des surfaces d'appui de parois longitudinales (44.1) et étant adaptées, pour un premier ensemble (45.1) d'entre elles, à appliquer à la première électrode (21) un potentiel électrique et, pour un deuxième ensemble (46.1) d'entre elles, à appliquer à l'anode (31) un potentiel électrique différent.

12. Dispositif électrochimique (1) selon la revendication 11, dans lequel la première plaque bipolaire (13.1) comporte une première (49.1) et une deuxième (50.1) lignes conductrices dites de reprise de contact s'étendant sur la face externe (420.1), et des premiers (47.1) et deuxièmes (48.1) vias conducteurs s'étendant entre la face externe (420.1) et la face interne (42i.1) dans les parois longitudinales (44.1), la première ligne conductrice (49.1) de reprise de contact étant adaptée à appliquer un potentiel électrique au premier ensemble (45.1) de lignes conductrices par des vias conducteurs (47.1), et la deuxième ligne conductrice (50.1) de reprise de contact étant adaptée à appliquer un potentiel électrique différent au deuxième ensemble (46.2) de lignes conductrices par les autres vias conducteurs (48.1).

13. Dispositif électrochimique (1) selon l'une quelconque des revendications précédentes, comportant un deuxième collecteur d'évacuation (15.2) en communication fluidique avec ladite deuxième face (19.2) et un deuxième circuit de distribution fluidique (18.2) en communication avec ledit deuxième collecteur d'évacuation (15.2), la deuxième électrode (22) étant adaptée à assurer une oxydation d'hydrogène circulant dans le deuxième circuit de distribution (18.2) ou étant adaptée à assurer une réduction de protons ayant diffusé au travers de la membrane électrolytique (19).

## Patentansprüche

1. Elektrochemische Vorrichtung (1), umfassend wenigstens eine elektrochemische Zelle (10), umfassend:
∘ eine Elektrodenmembran-Struktur (11), die aus einer elektrolytischen Protonentauscher-Membran (19), einer ersten Elektrode (21), die mit einer ersten Seite (19.1) der Membran (19) in Kontakt ist, und einer zweiten Elektrode (22), die mit einer zweiten Seite (19.2) gegenüber der Membran (19) in Kontakt ist, geformt ist,
∘ zwei bipolare Platten (13.1, 13.2), zwischen denen die Elektrodenmembran-Struktur (11) angeordnet ist, die durch wenigstens einen ersten Austragsauffang (15.1) durchquert sind, der in fluidischer Kommunikation mit der ersten Seite (19.1) der Membran (19) ist,
∘ wobei die Elektrodenmembran-Struktur (11) einen aktiven Bereich (2), der durch die ersten und zweiten Elektroden (21, 22) begrenzt ist, und einen Verbindungsbereich (3), der zwischen dem aktiven Bereich (2) und dem ersten Austragsauffang (15.1) angeordnet ist, umfasst;
**dadurch gekennzeichnet, dass** sie wenigstens einen Wasserstoffsensor (30) umfasst, umfassend:
∘ eine Anode (31), die in dem Verbindungsbereich (3) in Kontakt mit der ersten Seite (19.1) positioniert ist, und einen Katalysator umfasst, der geeignet ist, die Oxidation des Wasserstoffs zu gewährleisten, und eine Kathode (32) in Kontakt mit der zweiten Seite (19.2) und die gegenüber der Anode (31) angeordnet ist;
∘ eine Spannungsquelle (33), die zum Anwenden einer elektrischen Spannung zwischen der Anode (31) und der Kathode (32) durch eine elektrische Schaltung geeignet ist;
∘ einen Stromsensor (35), der an die Spannungsquelle (33) angeschlossen und geeignet ist, den elektrischen Strom zu messen, der in der elektrischen Schaltung fließt;
∘ eine Berechnungseinheit (36), die an den Stromsensor (35) angeschlossen und geeignet ist, das Vorhandensein von Wasserstoff an der ersten Seite (19.1) ausgehend von dem gemessenen Wert des elektrischen Stroms zu erfassen.

2. Elektrochemische Vorrichtung (1) nach Anspruch 1, bei der die Berechnungseinheit (36) zum Berechnen der an der Anode (31) oxidierten Wasserstoffmenge ausgehend von dem gemessenen Wert des elektrischen Stroms geeignet ist.

3. Elektrochemische Vorrichtung (1) nach Anspruch 1 oder 2, bei der die Berechnungseinheit (36) zum Berechnen der Wasserstoffmenge geeignet ist, die in dem Verbindungsbereich (3) an der ersten Seite (19.1) ausgehend von dem gemessenen Wert des elektrischen Stroms fließt.

4. Elektrochemische Vorrichtung (1) nach irgendeinem der Ansprüche 1 bis 3, bei der eine erste bipolare Platte (13.1) einen ersten fluidischen Verteilerkreislauf (18.1) in Kommunikation mit dem ersten Austragsauffang (15.1) und umfassend wenigstens einen der Anode (31) überlagerten Verteilerkanal umfasst.

5. Elektrochemische Vorrichtung (1) nach irgendeinem der Ansprüche 1 bis 4, bei der jede bipolare Platte (13.1, 13.2) aus wenigstens einem Blech geformt ist, das aus einem elektrisch leitenden Material realisiert ist.

6. Elektrochemische Vorrichtung (1) nach Anspruch 5, bei der die Anode (31) und die Kathode (32) von den bipolaren Platten (13.1, 13.2) elektrisch isoliert sind.

7. Elektrochemische Vorrichtung (1) nach Anspruch 5 oder 6, bei der die elektrolytische Membran (19) einen hervorstehenden Abschnitt (24), der auf einem Rand des Verbindungsbereichs (3) angeordnet ist, wobei die Spannungsquelle (33) eine erste Leiterbahn (34.1) umfasst, die mit der ersten Seite (19.1) der Membran (19) fest verbunden ist und die Anode (31) mit dem hervorstehenden Abschnitt (24) verbindet, und eine zweite Leiterbahn (34.2), die fest mit der zweiten Seite (19.2) verbunden ist und die Kathode (32) des hervorstehenden Abschnitts (24) verbindet, umfasst.

8. Elektrochemische Vorrichtung (1) nach Anspruch 7, bei der die erste und zweite Leiterbahn (34.1, 34.2) eine Dicke von weniger als oder gleich 10 µm aufweisen.

9. Elektrochemische Vorrichtung (1) nach irgendeinem der Ansprüche 1 bis 4, bei dem eine erste und eine zweite bipolare Platte (13.1, 13.2) geformt sind, jede in einer Platte (41.1, 41.2), die aus einem elektrisch isolierenden Material realisiert ist, und umfassend leitende Leitungen (45, 47, 49, 46, 48, 50), die derart angeordnet sind, dass sie jeweils die erste Elektrode (21) unabhängig von der Anode (31) und die zweite Elektrode (22) unabhängig von der Kathode (32) polarisieren.

10. Elektrochemische Vorrichtung (1) nach Anspruch 9, bei der die erste bipolare Platte (13.1) in einem Stück realisiert ist und eine innere Seite (42i.1) und eine äußere Seite (42e.1) aufweist, wobei die innere Seite (42i.1) Strukturierungen aufweist, die einen fluidischen Verteilerkreislauf (18.1) formen, wobei die erste bipolare Platte (13.1) zum Anwenden eines ersten elektrischen Potenzials auf die erste Elektrode (21) und eines von dem ersten Potenzial unterschiedlichen zweiten elektrischen Potenzials auf die Anode (31) geeignet ist.

11. Elektrochemische Vorrichtung (1) nach Anspruch 10, bei der das isolierende Material (41.1) der ersten bipolaren Platte (13.1) die innere Seite (42i.1) und die entgegengesetzte, äußere Seite (42e.1) definiert, wobei die innere Seite (42i.1) fluidische Verteilerkanäle umfasst, die in Längsrichtung zu zweit durch eine Längswand (44.1) getrennt sind, die mit der ersten Elektrode (21) oder der Anode (31) durch eine Stützfläche in Kontakt kommt, wobei sich elektrischleitende Leitungen (45.1, 46.1) an den Stützflächen von Längswänden (44.1) erstrecken und geeignet sind, für eine erste Gruppe (45.1) von ihnen ein elektrisches Potenzial auf die erste Elektrode (21) anzuwenden und für eine zweite Gruppe (46.1) von ihnen ein unterschiedliches elektrisches Potenzial auf die Anode (31) anzuwenden.

12. Elektrochemische Vorrichtung (1) nach Anspruch 11, bei der die erste bipolare Platte (13.1) eine erste (49.1) und eine zweite (50.1) leitende Leitung, bezeichnet als Kontaktwiederherstellung, die sich auf der äußeren Seite (42e.1) erstreckt, und erste (47.1) und zweite (48.1) leitende Lücken, die sich zwischen der äußeren Seite (42e.1) und der inneren Seite (42i.1) in den Längswänden (44.1) erstrecken, umfasst, wobei die erste leitende Leitung (49.1) zur Kontaktwiederherstellung geeignet ist, um ein elektrisches Potenzial auf die erste Gruppe (45.1) von leitenden Leitungen durch leitende Lücken (47.1) anzuwenden, und die zweite leitende Leitung (50.1) zur Kontaktwiederherstellung geeignet ist, ein elektrisches Potenzial anzuwenden, das von der zweiten Gruppe (46.2) von leitenden Leitungen durch die anderen leitenden Lücken (48.1) unterschiedlich ist.

13. Elektrochemische Vorrichtung (1) nach irgendeinem der voranstehenden Ansprüche, umfassend einen zweiten Austragsauffang (15.2) in fluidischer Verbindung mit der zweiten Seite (19.2) und einen zweiten fluidischen Verteilerkreislauf (18.2) in Verbindung mit dem zweiten Austragsauffang (15.2), wobei die zweite Elektrode (22) geeignet ist, um eine Wasserstoffoxidation zu gewährleisten, die in dem zweiten Verteilerkreislauf (18.2) fließt oder geeignet ist, eine Protonenreduzierung zu gewährleisten, die durch die elektrolytische Membran (19) diffundiert sind.

## Claims

1. Electrochemical device (1), comprising at least one electrochemical cell (10), comprising:
∘ a membrane electrode assembly (11) formed of an electrolytic proton-exchange membrane (19), of a first electrode (21) in contact with a first face (19.1) of the membrane (19), and of a second electrode (22) in contact with a second, opposite face (19.2) of the membrane (19),
∘ two bipolar plates (13.1, 13.2), between which the membrane electrode assembly (11) is located, at least one first discharge manifold (15.1) passing through said bipolar plates and in fluidic communication with the first face (19.1) of the membrane (19),
∘ the membrane electrode assembly (11) comprising an active zone (2) delimited by the first and second electrodes (21, 22), and a connection zone (3) located between the active zone (2) and the first discharge manifold (15.1);
**characterized in that** it comprises at least one hydrogen sensor (30), comprising:
∘ an anode (31) positioned in the connection zone (3) in contact with the first face (19.1) and including a catalyst suitable for ensuring the oxidation of the hydrogen, and a cathode (32) in contact with the second face (19.2) and located opposite the anode (31);
∘ a voltage source (33) suitable for applying a voltage between the anode (31) and the cathode (32) via an electric circuit;
∘ a current sensor (35), connected to the voltage source (33), suitable for measuring the electric current flowing in the electric circuit;
∘ a computing unit (36), connected to the current sensor (35), suitable for detecting the presence of hydrogen on the first face (19.1) from the measured value of the electric current.

2. Electrochemical device (1) according to Claim 1, in which the computing unit (36) is suitable for calculating the amount of hydrogen oxidized at the anode (31) from the measured value of the electric current.

3. Electrochemical device (1) according to Claim 1 or 2, in which the computing unit (36) is suitable for calculating the amount of hydrogen circulating in the connection zone (3) on the first face (19.1), from the measured value of the electric current.

4. Electrochemical device (1) according to any one of Claims 1 to 3, in which a first bipolar plate (13.1) comprises a first fluid distribution circuit (18.1) in communication with said first discharge manifold (15.1) and comprising at least one distribution channel superimposed on the anode (31).

5. Electrochemical device (1) according to any one of Claims 1 to 4, in which each bipolar plate (13.1, 13.2) is formed of at least one sheet made of an electrically conductive material.

6. Electrochemical device (1) according to Claim 5, in which the anode (31) and the cathode (32) are electrically insulated from the bipolar plates (13.1, 13.2).

7. Electrochemical device (1) according to Claim 5 or 6, in which the electrolytic membrane (19) comprises a protruding portion (24) located in an edge of the connection zone (3), the voltage source (33) comprising a first conductive track (34.1) attached to the first face (19.1) of the membrane (19) and connecting the anode (31) to the protruding portion (24) and a second conductive track (34.2) attached to the second face (19.2) and connecting the cathode (32) to the protruding portion (24).

8. Electrochemical device (1) according to Claim 7, in which the first and second conductive tracks (34.1, 34.2) have a thickness less than or equal to 10 µm.

9. Electrochemical device (1) according to any one of Claims 1 to 4, in which a first and a second bipolar plate (13.1, 13.2) are each formed of a plate (41.1, 41.2) made of an electrically insulating material, and comprising conductive lines (45, 47, 49; 46, 48, 50) arranged so as to polarize, respectively, the first electrode (21) independently of the anode (31), and the second electrode (22) independently of the cathode (32).

10. Electrochemical device (1) according to Claim 9, in which the first bipolar plate (13.1) is made in one piece, and has an inner face (42i.1) and an outer face (42e.1), the inner face (42i.1) having structurings that form a fluid distribution circuit (18.1), the first bipolar plate (13.1) being suitable for applying a first electric potential to the first electrode (21) and a second electric potential to the anode (31) that is different from the first potential.

11. Electrochemical device (1) according to Claim 10, in which the insulating material (41.1) of the first bipolar plate (13.1) defines the inner face (42i.1) and the opposite outer face (42e.1), the inner face (42i.1) comprising fluid distribution channels that are separated longitudinally in twos by a longitudinal wall (44.1) that comes into contact with the first electrode (21) or with the anode (31) via a bearing surface, electrically conductive lines (45.1, 46.1) extending on the bearing surfaces of longitudinal walls (44.1) and being suitable, for a first set (45.1) thereof, for applying an electric potential to the first electrode (21) and, for a second set (46.1) thereof, for applying a different electric potential to the anode (31).

12. Electrochemical device (1) according to Claim 11, in which the first bipolar plate (13.1) comprises a first conductive line (49.1) and a second conductive line (50.1), referred to as contact-making lines, extending on the outer face (42e.1), and first (47.1) and second (48.1) conductive vias extending between the outer face (42e.1) and the inner face (42i.1) in the longitudinal walls (44.1), the first contact-making conductive line (49.1) being suitable for applying an electric potential to the first set (45.1) of conductive lines through conductive vias (47.1), and the second contact-making conductive line (50.1) being suitable for applying a different electric potential to the second set (46.2) of conductive lines through the other conductive vias (48.1).

13. Electrochemical device (1) according to any one of the preceding claims, comprising a second discharge manifold (15.2) in fluidic communication with said second face (19.2) and a second fluid distribution circuit (18.2) in communication with said second discharge manifold (15.2), the second electrode (22) being suitable for ensuring an oxidation of hydrogen circulating in the second distribution circuit (18.2) or being suitable for ensuring a reduction of protons that have diffused across the electrolytic membrane (19).
